(19) Europäisches Patentamt

European Patent Office

Office européen des brevets

(11) Publication number: **0 362 915**
**A2**

(12) **EUROPEAN PATENT APPLICATION**

(21) Application number: 89202258.3

(22) Date of filing: 07.09.89

(51) Int. Cl.⁵: **C07K 7/08 , A61K 39/21 ,**
**G01N 33/569**

(30) Priority: 09.09.88 NL 8802217

(43) Date of publication of application:
**11.04.90 Bulletin 90/15**

(84) Designated Contracting States:
**AT BE CH DE ES FR GB GR IT LI NL SE**

(71) Applicant: **AKZO N.V.**
**Velperweg 76**
**NL-6824 BM Arnhem(NL)**

(72) Inventor: **Hellings, Jan Albert**
**Thorbeckestraat 20**
**NL-5344 SN Oss(NL)**
Inventor: **Schalken, Johannes Jacobus**
**De Kuiper 7**
**NL-5283 MK Boxtel(NL)**
Inventor: **Sprengers, Erik Dagobert**
**Dordognelaan 29**
**NL-5627 HB Eindhoven(NL)**

(74) Representative: **Hermans, Franciscus G.M. et**
**al**
**Patent Department AKZO N.V. Pharma**
**Division P.O. Box 20**
**NL-5340 BH Oss(NL)**

(54) Synthetic polypeptides immunochemically reactive with HIV-antibodies.

(57) The invention concerns synthetic polypeptides which react immunochemically with antibodies directed against HIV. A method for the detection of HIV or anti-HIV in a test fluid, an immunochemical reagent and a test kit to be used when applying said detection methods belong to the invention.

EP 0 362 915 A2

## Synthetic polypeptides immunochemically reactive with HIV-antibodies.

The invention relates to synthetic polypeptides which react immunochemically with antibodies directed against HIV.

The invention also relates to a method for the detection of HIV or anti-HIV in a test fluid, and also to an immunochemical reagent and a test kit to be used when applying said detection methods.

The human immunodeficiency virus type 1 (HIV-1)) is generally regarded as the pathogen of the "acquired immunodeficiency syndrome" (AIDS).

In recent years the HIV has spread enormously and it is expected that it will spread even further in the future. The treatment of infected individuals, but even more the prevention of the transmission to uninfected individuals, is of the greatest importance. Despite the great deal of research which has already been carried out into the origin and the spread of the virus and the AIDS disease, many problems are still waiting for a solution. One of these problems is the lack of very specific and sensitive methods to enable an early diagnosis of the infection to be made.

The general structure of HIV consists of a ribonucleo-protein core surrounded by a lipid-containing covering or envelope. The genome of HIV consists of three main regions: the gag and env regions which contain information for structural proteins and the pol region which contains information for the virus RNA-dependent DNA polymerase (reverse transcriptase), protease and endonuclease. The protein products of the pol region of HIV are three enzyme-active proteins of 66/51 kilodalton (p66/p51), 32 kilodalton (p32) and 34 kilodalton (p34) respectively.

Although these proteins of HIV-1 are the subject of a great deal of research, the immuno-dominant epitopes of these proteins have still not been completely localized.

However, for the development of a specific and sensitive method to enable a reliable diagnosis to be made in various phases of the infection with HIV it is of great importance to identify immuno-dominant viral epitopes of this type.

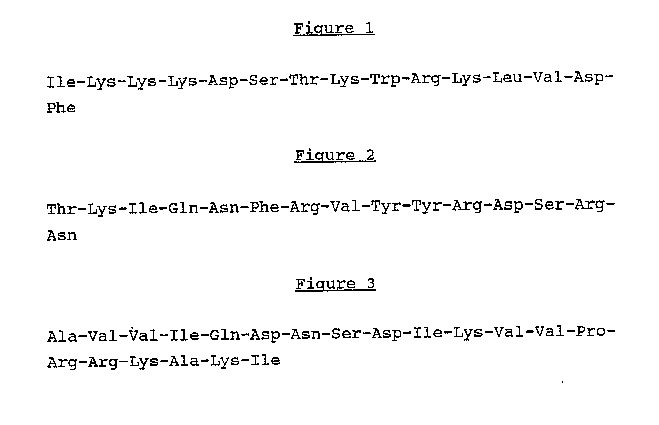

Three synthetic polypeptides have now been found with, respectively, 15, 15 and 20 amino acids and an amino acid sequence as shown in Figure 1, Figure 2 and Figure 3 respectively which are exceptionally immuno-chemically reactive with HIV-antibodies.

The invention also includes fragments of the said pentadeca-, pentadeca- and eicosa-peptides which are still immunochemically reactive with HIV-antibodies and also synthetic polypeptides which contain the said pentadeca-, pentadeca- or eicosa-peptides as an essential constituent, or fragments thereof which are immunochemically reactive with HIV-antibodies.

The invention also relates to an immunochemical reagent, which reagent contains at least one of the synthetic peptides according to the invention.

The invention also comprises a method for the detection of antibodies directed against HIV in a test fluid, with which use is made of at least one of the synthetic peptides according to the invention.

The invention also relates to a method for the detection of HIV in a test fluid, using at least one of the synthetic peptides according to the invention.

The invention also relates to a test kit to be used in an immuno-assay, this test kit containing at least one immunochemical reagent according to the invention.

The synthetic peptides mentioned above are found to be particularly suitable for use in a diagnostic method for determining the presence of HIV or HIV-antibodies in a test fluid.

In contrast to the native HIV, the peptides according to the invention have the great advantage that these are of a safe non-infectious origin.

The preparation of the synthetic peptides according to the invention is effected by means of one of the known organic chemical methods for peptide synthesis or with the aid of recombinant DNA techniques. This latter method involves the preparation of the desired polypeptide by means of bringing to expression a recombinant polynucleotide with a polynucleotide sequence which is coding for one or more of the polypeptides in question in a suitable micro-organism as host.

The organic chemical methods for peptide synthesis are considered to include the coupling of the required aminoacids by means of a condensation reaction, either in homogeneous phase or with the aid of a so-called solid phase.

The condensation reaction in homogeneous phase can be carried out as follows:

a) condensation of a compound (amino acid, peptide) with a free carboxyl group and protected other reactive groups with a compound (amino acid, peptide) with a free amino group and protected other reactive groups, in the presence of a condensation agent,

b) condensation of a compound (amino acid, peptide) with an activated carboxyl group and free or protected other reactive groups with a compound (amino acid, peptide) with a free amino group and free or protected other reactive groups.

Activation of the carboxyl group can take place, inter alia, by converting the carboxyl group to an acid halide, an azide, anhydride, imidazolide or an activated ester, such as the N-hydroxy-succinimide, N-hydroxy-benztriazole or p-nitrophenyl ester.

The most common methods for the above condensation reactions are: the carbodiimide method, the azide method, the mixed anhydride method and the method using activated esters, such as described in "The Peptides, Analysis, Synthesis, Biology" Vols. 1-3 (Ed. Gross, E. and Meienhofer, J.) 1979, 1980, 1981 (Academic Press).

Preparation of the abovementioned peptides according to the invention is, however, also possible using the "solid phase" method of Merrifield, described in J. Amer. Chem. Soc. 85, 2149 (1963). The coupling of the aminoacids of the peptides to be prepared preferably starts from the carboxyl end side. For this method a solid phase is needed on which there are reactive groups or on which such groups can be introduced. This can be, for example, a copolymer of benzene and divinylbenzene with reactive chloromethyl groups, or a polymeric solid phase rendered reactive with hydroxymethyl or benzylamine.

A particularly suitable solid phase is, for example, the p-alkoxybenzyl alcohol resin (4-hydroxymethyl, phenoxy-methyl-copolystyrene-1% divinylbenzene resin), described by Wang (1974) J. Am. Chem. Soc. 95, 1328. After synthesis the peptides can be split from this solid phase under mild conditions.

After synthesis of the desired aminoacid sequence, detaching of the peptide from the resin follows with, for example, with trifluoromethanesulphonic acid or with methanesulphonic acid dissolved in trifluoroacetic acid. The peptide can also be removed from the carrier by transesterification with a lower alcohol, preferably methanol or ethanol, in which case a lower alkyl ester of the peptide is formed directly. Likewise, splitting with the aid of ammonia gives the amide of a peptide according to the invention.

The reactive groups which may not participate in the condensation reaction are, as stated, effectively protected by groups which can be removed again very easily by hydrolysis with the aid of acid or reduction. Thus, a carboxyl group can be effectively protected by, for example, esterification with methanol, ethanol, tertiary butanol, benzyl alcohol or p-nitrobenzyl alcohol.

Groups which can effectively protect an amino group are the ethoxycarbonyl, benzyloxycarbonyl, t-butoxycarbonyl or p-methoxy-benzyloxycarbonyl group, or an acid group derived from a sulphonic acid, such as the benzene-sulphonyl or p-toluenesulphonyl group, but other groups can also be used, such as substituted or unsubstituted aryl or aralkyl groups, for example benzyl and triphenylmethyl, or groups such as ortho-nitrophenylsulphenyl and 2-benzoyl-1-methylvinyl. A particularly suitable α-amino-protective group is, for example, the base-sensitive 9-fluorenyl-methoxycarbonyl (Fmoc) group [Carpino & Han (1970) J. Amer. Chem. Soc. 92, 5748].

It is necessary also to protect the ε-amino group of lysine and advisable for the guanidine group of arginine. Customary protective groups in this connection are a Boc-group for lysine and a Pms-, Mbs-group or Mtr-group for arginine.

The protective groups can be split off by various conventional methods, depending on the nature of the particular group, for example with the aid of trifluoroacetic acid or by mild reduction, for example with hydrogen and a catalyst, such as palladium, or with HBr in glacial acetic acid.

As already indicated above, the peptide according to the invention can likewise be prepared with the aid of recombinant DNA techniques. This possibility is of importance particularly when the peptide is incorporated in a repeating sequence ("in tandem") or when the peptide can be prepared as a constituent of a (much larger) protein or polypeptide. This type of preparation of the peptide therefore likewise falls within the scope of the invention. For this purpose, as a constituent of a recombinant DNA, a polynucleotide is used which codes for the peptide according to the invention and which, furthermore, is substantially free from polynucleotide segments, which in the naturally occurring HIV genome flank the polynucleotide sequence indicated above.

A polynucleotide of this type, which is coding for the peptide according to the invention, and a recombinant DNA in which this polynucleotide is incorporated likewise fall within the scope of the invention.

Without this specifically being incorporated in the claims, it is self-evident that one or more aminoacids in the peptides according to the invention can be replaced by other aminoacids such as is indicated in the Los Alamos Data Bank (AIDS virus sequence data base).

In addition the functional derivatives of these peptides, by which are meant in the main:

   a) acid addition salts of the peptides;

   (b) amides of the peptides and specifically the C-terminal amides;

   (c) esters and specifically C-terminal esters and

   (d) N-acyl derivatives, specifically N-terminal acyl derivatives and in particular N-acetyl derivatives, are also considered as peptides according to the invention.

The term "immunochemical reagent" signifies that the synthetic peptides according to the invention are bonded to a suitable support or are provided with a labelling substance.

The supports which can be used are, for example, the inner wall of a microtest well, a tube or capillary, a membrane, filter, test strip or the surface of a particle such as, for example, a latex particle, an erythrocyte, a dye sol, a metal sol or metal compound as sol particle.

Labelling substances which can be used are, inter alia, a radioactive isotope, a fluorescent compound, an enzyme, a dye sol, metal sol or metal compound as sol particle.

In a method for the detection of antibodies directed against HIV in a test fluid, an immunochemical reagent according to the invention is used, which reagent is brought into contact with the test fluid, and the presence of immune complexes, formed between the peptide and antibodies in the test fluid, which are reactive with the peptide, is detected and from this the presence of HIV-antibodies in the test fluid is determined.

The immunochemical reaction which must take place when using these detection methods is preferably a sandwich reaction, an agglutination reaction, a competition reaction or an inhibition reaction.

For the detection of HIV in a test fluid where use is made of anti-HIV, an immunochemical reagent according to the invention is used, this reagent being brought into contact with the test fluid and the presence of immune complexes formed between peptide and anti-HIV being detected and, from this, the presence of HIV in a test fluid being determined.

A particularly suitable method for the detection of HIV in a test fluid which can be used is a competition reaction between a synthetic peptide according to the invention provided with a labelling substance and a HIV-antigen (present in the test fluid) for a bonding site on the antibody directed against HIV which is bonded to a solid support.

A test kit according to the invention must contain, as an essential constituent, an immunochemical reagent such as described above. For carrying out a sandwich reaction, the test kit can consist, for example, of the synthetic peptide according to the invention bonded to a solid support, for example the inner wall of a microtest well, it being possible to use either a labelled synthetic peptide according to the invention or a labelled anti-antibody.

For carrying out a competition reaction, the test kit can consist of the synthetic peptide according to the invention bonded to a solid support, a labelled antibody directed against HIV then being used to compete with anti-HIV in a test fluid.

In an agglutination reaction an immunochemical reagent which consists of a synthetic peptide according to the invention bonded to particles or sols must be brought directly into contact with the test fluid in which the antibodies directed against HIV which are to be detected are present.

Another embodiment of a test kit is, for example, the use of a labelled synthetic peptide according to the invention as immunochemical reagent in a competition reaction with a HIV antigen to be detected for a bonding site on the antibody directed against HIV, which is bonded to a solid support.

## Example I

The pentadecapeptide with the sequence as shown in figure 2 (pol-peptide HIV-1; 945-959) was prepared by stepwise solid phase peptide synthesis. The synthesis was carried out using a VEGA Coupler 250 C automated peptide synthesizer, employing a p-benzyloxybenzyl alcohol resin (Wang-resin; 0.6-0.7 mmoles/g, Bachem AG, Switzerland) and $N^{\alpha}$-Fmoc-protected (Fmoc, 9-fluorenylmethyloxycarbonyl) amino acids.

The synthesis started by the coupling of Fmoc-Asn(Ddm)-OH(Ddm, 4,4'-dimethoxydiphenylmethyl) to the resin using DCC (dicyclohexylcarbodiimide, 1 equivalent), HOBt (1-hydroxybenzotriazole, 2 equivalents) and DMAP (N,N-dimethylaminopyridine, 1 equivalent) in DMF-dichloromethane (1:1, vol/vol) at 4 $^{\circ}$C for 18 hours. Unreacted alcohol functions on the resin were subsequently blocked by benzoylation using benzoylchloride-pyridine (1:1, vol/vol) for 2 hours.

The resulting Fmoc-Asn(Ddm)-resin (0.38 mmoles/g) was successively treated with 25% piperidine in DMF for 3 min and 10 min, respectively, in order to remove the Fmoc-group. The protected pentadecapeptide was then prepared on the H-Asn(Ddm)-resin by successive coupling steps of the Fmoc-amino acids, as dictated by the amino acid sequence. The following side chain protecting groups were used: -Mtr (4-methoxy, 2,3,6-trimethylbenzene sulfonyl) for Arg; -tBu (tert.butyl) for Ser, Thr and Tyr; -OtBu (tert.butyloxy) for Asp and -Boc (t.butyloxycarbonyl) for Lys.

Each coupling step was performed using 3 equivalents each of Fmoc-amino acid, DCC and HOBt in 6-7 ml of DMF per gram resin for 2 hours, followed by 3 washings (one min each) with ethanol, DMF and dichloromethane. Completeness of the coupling reaction was monitored by the ninhydrine test of Kaiser (Anal.Biochem. 34, 595-598, 1970). A positive ninhydrine reaction was observed following coupling of Arg[958], Val[952], Arg[951] Ile[947] and Thr[945].

In each case the coupling reaction was repeated once, followed by capping of any remaining free amino groups by reaction with acetic anhydride-pyridine (2:1, vol/vol) for 10 min and subsequent washings with DMF and dichloromethane (1 min each), respectively.

After each synthesis cycle the Fmoc-protecting group was removed by treatment with 25% piperidine in DMF as described above.

After completion of the synthesis and following removal of the N-terminal Fmoc-group, the resulting protected pentadecapeptide resin was treated for 3 hours at room temperature in a mixture of trifluoro acetic acid-methanesulfonic acid and anisole (9:1:0.5, vol/vol) in order to effect release of the peptide from the resin with simultaneous removal of all protecting groups. The crude peptide was isolated following precipitation upon addition of diethylether to the reaction mixture.

The pentadecapeptide was purified by column chromatography on silica-60 in the solvent system butanol-1-pyridine-acetic acid-water (4:2:2:1, vol/vol). The amino acid analysis of the peptide was in agreement with the sequence synthesized.

Example II

The synthetic pentadecapeptide according to Figure 1 was synthesized by the solid phase method as described for the amino acid sequence in Example I.

Example III

The synthetic eicosapeptide according to Figure 3 was synthesized by the solid phase method as described for the amino acid sequence in Example I.

Example IV

The synthetic pentadecapeptide with the aminoacid sequence as shown in Figure 2 (polpeptide HIV-1, BRU, 945-959: aminoacid numbering according to the Los Alamos Data Bank (AIDS virus sequence database, Los Alamos National Laboratory, Theoretical Division, Los Alamos) was dissolved to 1 μg/ml in 0.05 M NaHCO₃ pH 9.6. For bonding the peptide to the microtiter plate, this solution was incubated overnight at 4° C in an 8 x 12 microtiter plate, 135 μl/well. The wells were then sucked empty and the residual bonding sites were blocked with a solution of phosphate-buffered physiological saline solution (PBS) with 5% skimmed milk powder and 4% normal goat serum for 1 hour at room temperature, 250 μl/well. The plates were then emptied and dried overnight at room temperature over silica gel.

For the determination of antibodies against HIV, the serum sample to be tested was diluted in PBS, pipetted (100 μl/well) and incubated for half an hour at 37° C. The plate was then washed 4 times with PBS, 0.05% Tween-20⁽ᴿ⁾. A solution of goat anti-human immunoglobulin labelled with peroxidase was then pipetted (100 μl/well) and incubated for half an hour at 37° C. The plate was then washed 4 times with PBS, 0.05% Tween-20⁽ᴿ⁾. As substrate for the peroxidase, a solution (100 μl/well) of urea peroxide (0.5 mg/ml) with orthophenylenediamine (OPD; 1.6 mg/ml) was then introduced into the wells and incubated at room temperature in the absence of light. The reaction was stopped after 30 minutes by adding 100 μl/well of 1 M H₂SO₄. The yellow colour was read off at 492 nm with the aid of an Organon Teknika microelisa reader. With anti-HIV positive sera extinctions of 0.15 to 0.60 were measured, while the extinction value in the case of the use of normal control human serum is 0.07.

**Claims**

1. Synthetic pentadecapeptide with aminoacid sequence as indicated in Figure 1 and fragments thereof which are immunochemically reactive with HIV-antibodies and a synthetic polypeptide, which contains the said pentadecapeptide as an essential constituent, or fragments thereof which are immunochemically reactive with HIV-antibodies.

2. Immunochemical reagent which contains a synthetic peptide according to Claim 1.

3. Synthetic pentadecapeptide with aminoacid sequence as indicated in Figure 2 and fragments thereof which are immunochemically reactive with HIV-antibodies and a synthetic polypeptide, which contains the said pentadecapeptide as an essential constituent, or fragments thereof which are immunochemically reactive with HIV-antibodies.

4. Immunochemical reagent which contains a synthetic peptide according to Claim 3.

5. Synthetic eicosapeptide with aminoacid sequence as indicated in Figure 3 and fragments thereof which are immunochemically reactive with HIV-antibodies and a synthetic polypeptide, which contains the said eicosapeptide as an essential constituent, or fragments thereof which are immunochemically reactive with HIV-antibodies.

6. Immunochemical reagent which contains a synthetic peptide according to Claim 5.

7. Method for the detection of antibodies directed against HIV in a test fluid, characterized in that an immunochemical reagent according to Claim 2, 4 or 6 is used, which reagent is brought into contact with the test fluid and the presence of immune complexes formed between the peptide and antibodies in the test fluid, which are reactive with the peptide, is detected and from this the presence of HIV-antibodies in the test fluid is determined.

8. Method for the detection of HIV in a test fluid, characterized in that an immunochemical reagent according to Claim 2, 4 or 6 is used, which reagent is brought into contact with the test fluid and the presence of immune complexes formed between peptide and anti-HIV is detected and from this the presence of HIV in the test fluid is determined.

9. Test kit to be used in an immuno-assay, at least containing one immunochemical reagent according to Claim 2, 4 or 6.

## Figure 1

Ile-Lys-Lys-Lys-Asp-Ser-Thr-Lys-Trp-Arg-Lys-Leu-Val-Asp-
Phe

## Figure 2

Thr-Lys-Ile-Gln-Asn-Phe-Arg-Val-Tyr-Tyr-Arg-Asp-Ser-Arg-
Asn

## Figure 3

Ala-Val-Val-Ile-Gln-Asp-Asn-Ser-Asp-Ile-Lys-Val-Val-Pro-
Arg-Arg-Lys-Ala-Lys-Ile